# EUROPEAN PATENT SPECIFICATION

(11) **EP 3 335 642 B1**
(45) Date of publication and mention of the grant of the patent: **20.10.2021**
(21) Application number: 17169003.5
(22) Date of filing: 31.10.2012
(51) Int. Cl.: A61B 17/04, A61B 17/06

(54) **APPARATUS FOR FORMING A SELF-LOCKING ADJUSTABLE LOOP**
VORRICHTUNG ZUM FORMEN EINER SELBSTSICHERNDEN, EINSTELLBAREN SCHLINGE
APPAREIL POUR FORMER UNE BOUCLE AJUSTABLE À VERROUILLAGE AUTOMATIQUE

(30) Priority: 03.11.2011 US 201113288459; 03.11.2011 US 201113288463
(43) Date of publication of application: 20.06.2018
(62) Divisional of application: 12791902.5
(73) Proprietor: Biomet Sports Medicine, LLC, Warsaw, Indiana 46582 (US)
(72) Inventor: DENHAM, Gregory J., Warsaw, IN Indiana 46582 (US); STONE, Kevin T., Winona Lake, IN Indiana 46590 (US); WAGNER, Zachary, Noblesville, IN Indiana 46060 (US)
(74) Representative: Mays, Julie

(56) References cited:
- US-A- 330 087
- US-A- 5 577 299
- US-A- 5 699 657
- US-A1- 2003 065 402
- US-A1- 2009 318 961
- US-A1- 2011 264 141

## Description

### FIELD

The present disclosure relates generally to an apparatus for forming a self-locking adjustable loop.

### BACKGROUND

The statements in this section merely provide background information related to the present disclosure and may not constitute prior art.

Tears caused by trauma or disease in soft tissue, such as cartilage, ligament, or muscle, can be repaired by suturing. Various repair techniques and devices have been developed for facilitating suturing that include the use of an intermediate member to facilitate coupling the suture to the soft tissue and are effective for their intended purposes. Nevertheless, there is still a need in the relevant art for tissue repair techniques and associated suture constructs for facilitating suturing without requiring the use of such intermediate members. Document US5 699 657 discloses families of slipping braid splices and the method of forming such splices.

### SUMMARY

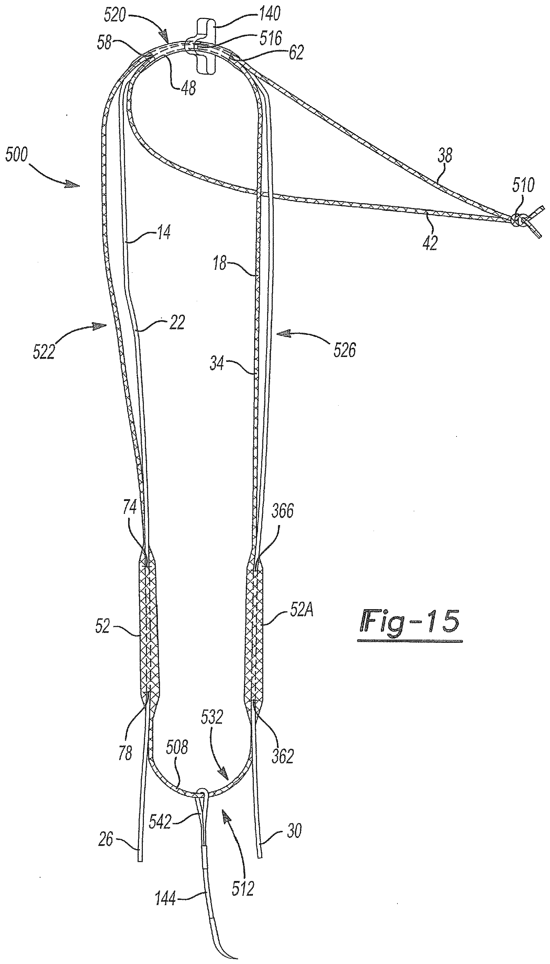

The present invention is disclosed in the appended set of claims and is illustrated in figure 15. This section provides a general summary of the disclosure, and is not a comprehensive disclosure of its full scope or all of its features.

In one aspect, an apparatus for use in surgical implantation is provided in accordance with the present teachings. The apparatus can include a first flexible member and a second flexible member. The first flexible member can have a first end, a second end and a first body extending therebetween, where the first body defines a first passage portion. The second flexible member can have a first end, a second end and a second body extending therebetween, where the second body defines a second passage portion. The first end of the first flexible member can pass into and through the second passage portion in a first direction such that the first end of the first flexible member extends outside of the second passage portion. The first end of the second flexible member can pass into and through the first passage portion in a second direction such that the first end of the second flexible member extends outside of the first passage portion to form a self-locking adjustable flexible member construct. Applying tension to the first ends of the first and second flexible members can draw the first and second passage portions and corresponding second ends toward each other.

In another aspect, an apparatus for use in surgical implantation is provided in accordance with the present teachings. The apparatus can include first and second sutures. The first suture can have a first end, a second end and a first body extending therebetween, where the first body defines a first passage portion. The second suture can have a first end, a second end and a second body extending therebetween, where the second body defines a second passage portion. The first end of the first suture can pass into and through the second passage portion such that the first end of the first suture extends outside of the second passage portion, and the second end of the first suture can pass into and through the second passage portion in a direction opposite the first end of the first suture so as to form a first self-locking adjustable loop. The first end of the second suture can pass into and through the first passage portion such that the first end of the second suture extends outside of the first passage portion, and the second end of the second suture can pass into and through the first passage portion in a direction opposite the first end of the second suture to form a second self-locking adjustable loop. Applying tension to the first and second ends of the first and second sutures can reduce a size of the first and second adjustable loops.

In yet another aspect, a method of using a flexible member construct in a surgical procedure is provided in accordance with the present teachings. The method can include forming first and second bores in a bone and carrying first and second flexible anchors into the respective first and second bores, where the first and second flexible anchors can each include an internal passage slidably coupled to an adjustable suture construct. The adjustable suture construct can have first and second cooperating self-locking adjustable loops formed from first and second sutures. At least one of the first and second flexible anchors can be positioned through soft tissue. A shape of the first and second flexible anchors can be changed from a first profile to a second profile to retain the flexible anchors in the respective bores. Tension can be applied to ends of the first and second sutures of the adjustable suture construct to reduce a size of the first and second self-locking adjustable loops and secure the soft tissue relative to the first and second flexible anchors and the bone.

In the present invention, an apparatus for use in surgical implantation is provided in accordance with the present embodiments. The apparatus includes a first flexible member and a second flexible member. The first flexible member has a first end, a second end and a first body extending therebetween, where the first body defines a first passage portion. The second flexible member has a first end, a second end and a second body extending therebetween, where the second body defines second and third passage portions. The first end of the first flexible member passes into and through the second passage portion such that the first end extends outside of the second passage portion, and the second end of the first flexible member passes into and through the third passage portion such that the second end extends outside of the third passage portion. The first and second ends of the second flexible member pass into and through the first passage portion of the first flexible member to form a self-locking adjustable flexible member construct.

In another aspect, a method of using an adjustable flexible member construct in a surgical procedure is provided in accordance with the present teachings. The method can include passing a first end of a self-locking adjustable flexible member construct through soft tissue, where the flexible member construct has an adjustable portion formed from first and second flexible members coupled to and selectively movable relative to each other. A bore can be formed in a bone and an anchor member coupled to a second opposite end of the flexible member construct can be passed into the bore. Tension can be applied to first and second ends of the first flexible member extending from the anchor member and through the soft tissue. Tension can be applied to first and second ends of the second flexible member slidably extending from the anchor member to reduce a size of the adjustable portion of the flexible member construct and draw the soft tissue along and relative to the first flexible member toward the anchor member to automatically maintain the reduced size of the self-locking adjustable flexible member construct under tension.

In yet another aspect, a method of using a flexible member construct in a surgical procedure is provided in accordance with the present teachings. The method can include passing a self-locking adjustable suture construct through soft tissue, where the suture construct can have a first suture defining a first passage portion between first and second ends and a second suture defining second and third passage portions between third and fourth ends. The first and second ends can pass through the respective second and third passage portions at a first end of the suture construct and the third and fourth ends passing through the first passage portion at a second opposite end of the suture construct. The suture construct can have an adjustable length portion extending between the first passage portion and the second and third passage portions. The first end of the suture construct being passed though the soft tissue. A fixed length portion of the second suture extending between the second and third passage portions can be positioned over a portion of the soft tissue. A bore can be formed in a bone and an anchor member slidably coupled to the first passage portion can be passed into the bore. Tension can be applied to the first and second ends of the first suture extending from the soft tissue. Tension can be applied to the third and fourth ends of the second suture slidably extending from the anchor member while maintaining tension in the first suture to reduce a size of the adjustable portion and draw the soft tissue along and relative to the first suture toward the anchor member to automatically maintain the reduced size of the suture construct under tension via the first, second and third passage portions.

Further areas of applicability will become apparent from the description provided and drawings herein. The description and specific examples in this summary are intended for purposes of illustration only and are not intended to limit the scope of the present disclosure.

### DRAWINGS

The present teachings will become more fully understood from the detailed description, the appended claims and the following drawings. The drawings are for illustrative purposes only and are not intended to limit the scope of the present disclosure.
Figure 1 depicts an exemplary adjustable flexible member construct formed from two separate flexible member strands according to the present teachings;
Figure 2 depicts an exemplary assembly configuration of the adjustable flexible member construct of Figure 1 having an exemplary fixation member coupled thereto according to the present teachings;
Figure 3 depicts an exemplary technique for securing soft tissue to bone using the adjustable flexible member construct of Figures 1 and 2 according to the present teachings;
Figure 4 depicts an exemplary assembly configuration of the adjustable flexible member construct of Figure 1 having an exemplary flexible anchor coupled thereto according to the present teachings;
Figure 5 depicts an exemplary technique for securing soft tissue to bone using the adjustable flexible member construct of Figure 4 according to the present teachings;
Figure 6 depicts an exemplary adjustable flexible member construct formed from two separate flexible member strands according to the present teachings;
Figure 7 depicts an exemplary assembly configuration of the adjustable flexible member construct of Figure 6 having a pair of exemplary flexible anchors coupled thereto according to the present teachings;
Figure 8 depicts an exemplary technique for securing soft tissue to bone using the adjustable flexible member construct of Figures 6 and 7 according to the present teachings;
Figure 9 depicts an exemplary adjustable flexible member construct formed from two separate flexible member strands according to the present teachings;
Figures 10-13 depict an exemplary adjustable flexible member construct and an exemplary technique for forming the same according to the present teachings;
Figure 14 depicts an exemplary technique for securing soft tissue to bone using the adjustable flexible member construct of Figure 13 according to the present teachings;
Figure 15 depicts an exemplary adjustable flexible member construct according to the present invention,
Figures 16-17 depict enlarged views of portions of the adjustable flexible member construct of Figure 15 according to the present invention,
Figures 18-20 depict an exemplary method of forming a portion of the adjustable flexible member construct of Figure 15 according to the present teachings;
Figures 21-24 depict an exemplary technique for coupling the adjustable flexible member construct of Figure 15 to soft tissue according to the present teachings; and
Figures 25-29 depict an exemplary technique for securing the soft tissue to bone using the adjustable flexible member construct of Figure 15 according to the present teachings.

### DETAILED DESCRIPTION

The following description is merely exemplary in nature and is in no way intended to limit the present disclosure, its application, or uses. It should be understood that throughout the drawings, corresponding reference numerals indicate like or corresponding parts and features. While the disclosure generally relates to apparatus and associated methods for forming self-locking adjustable loops of flexible member constructs that can be used in securing soft tissue to bone, such as a rotator cuff or distal bicep, the apparatus and methods of the present teachings can be used in connection with various other soft tissue fixation methods and/or other procedures where flexible member tensioning and securing of soft tissue is required.

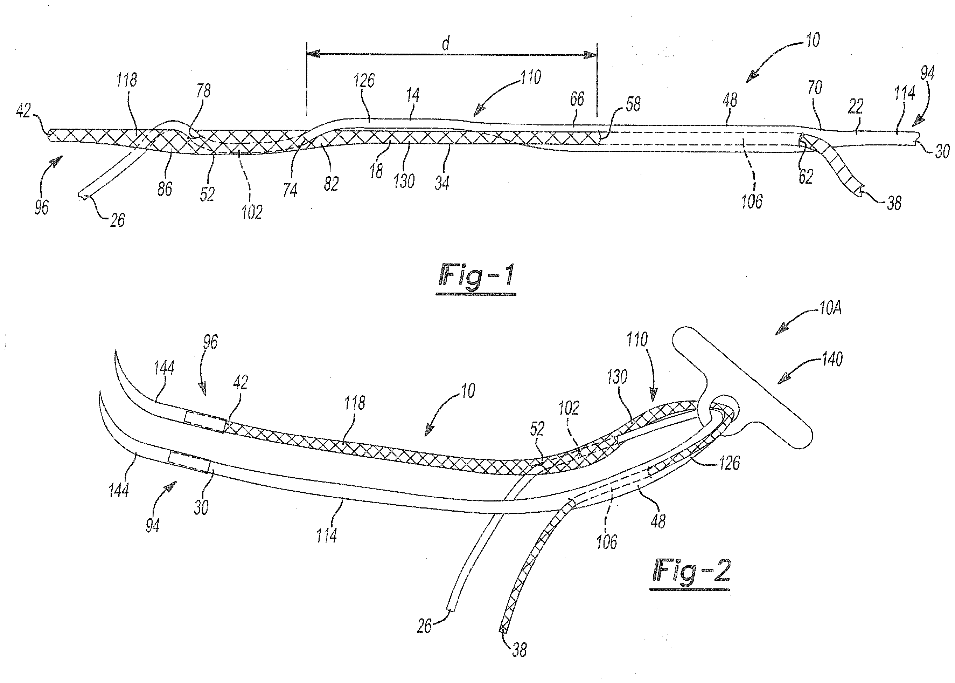

Referring to Figure 1, an adjustable flexible member construct 10 is provided according to various aspects of the present teachings. The adjustable flexible member construct 10 can be fashioned from first and second flexible members 14, 18 made of any biocompatible material including, but not limited to, non-resorbable polymers, such as polyethylene or polyester, resorbable polymers, and various combinations thereof. In various aspects, the flexible members 14, 18 can include a hollow material or core to allow for appropriate tensioning, as will be discussed herein. In various aspects, the flexible members 14, 18 can be sutures. In such aspects, the sutures can be a hollow or braided or multiple-filament braided suture structure having a hollow core. In various aspects, the sutures can be resorbable. In various aspects, the flexible members 14, 18 can define a substantially tubular hollow shape.

Flexible member 14 can include a body 22 extending between a first end 26 and a second end 30, and flexible member 18 can similarly include a body 34 extending between a first end 38 and a second end 42. The bodies 22, 34 can include respective formed first and second passage portions 48, 52, as also shown in Figures 1 and 2. In one exemplary aspect, the bodies 22, 34 can include an exterior surface and an interior surface defining an elongated passage between the respective first ends 26, 38 and second ends 30, 42. The bodies 22, 34 can define the passage portions 48, 52 as having a larger width than remaining portions of the bodies 22, 34. Alternatively, the passage portions 48, 52 can be formed initially to have the same width or diameter as the remaining portions of flexible member bodies 22, 34, later expanding in diameter during the construction process, which will be discussed below.

The first passage portion 48 can include first and second apertures 58, 62 positioned proximate first and second ends 66, 70 thereof. The second passage portion 52 can include third and fourth apertures 74, 78 positioned proximate third and fourth ends 82, 86 thereof, as shown in Figure 1. In various aspects, the apertures 58, 62, 74, 78 can be formed during a braiding process of flexible members 14, 18 as loose portions between pairs of fibers defining flexible members 14, 18, or can be formed during the construction process. Alternatively, ends of the flexible members 14, 18 can be pushed between individual fibers of the braided flexible members 14, 18 as will be discussed herein. The adjustable flexible member construct 10 can include a first end 94 and a second end 96. In one exemplary configuration, the first end 94 can be defined by the second end 30 of the first flexible member 14 and the second end 96 can be defined by the second end 42 of the second flexible member 18, as shown for example in Figures 1 and 2.

To form the adjustable flexible member construct 10, first end 26 of flexible member 14 can be passed through second passage portion 52 via third and fourth apertures 74, 78 such that a portion 102 of flexible member 14 following first end 26 extends through passage portion 52, as generally shown in Figure 1. In a similar manner, first end 38 of flexible member 18 can be passed through the first passage portion 48 via the first and second apertures 58, 62 such that a portion 106 of flexible member 18 following first end 38 extends through passage portion 48. This configuration can form an adjustable portion 110 of flexible member construct 10 between passage portions 48, 52, and can form fixed portions 114, 118 extending between respective passage portions 48, 52 and second ends 30, 42. In one exemplary aspect, adjustable portion 110 can include portions 126, 130 of respective flexible members 14, 18 extending between passage portions 48, 52, as shown for example in Figure 1.

With additional reference to Figure 2, adjustable flexible member construct 10 is shown in an assembly configuration 10A where an exemplary anchor member 140 is slidable coupled to flexible member construct 10. In the exemplary configuration illustrated, anchor member 140 is a toggle anchor configured to engage a boney structure and is coupled about the adjustable portion 110 of flexible member construct 10. The anchor member 140 can be, for example, a product sold by Biomet Sports Medicine, LLC under the name ToggleLoc^{™}. A further discussion of the anchor member 140 can be found in U.S. Pat. No. 7,601,165. As can also be seen in Figure 2, needles 144 or other suitable soft tissue piercing members can be coupled to second ends 30, 42 of flexible members 14, 18.

Adjustable flexible member construct 10 can provide an ability to secure the adjustable construct 10 directly to soft tissue, as well as provide an ability to reduce a size of only a portion of the adjustable construct 10 to thereby reduce an overall length of adjustable construct 10. In particular, by using the two separate flexible members 14, 18 coupled together via spaced apart passage portions 48, 52 in the manner discussed above, tension can be applied to first ends 26, 38 to reduce a length d of adjustable portion 110 relative to fixed portions 114, 118 and generally between passage portions 48, 52. In other words, tensioning first ends 26, 38 can draw the passage portions 48, 52 closer to one another thereby reducing a length of the portions 126, 130 (that form adjustable portion 110) and thus reduce the overall length of adjustable flexible member construct 10 without changing a length of fixed portions 114, 118.

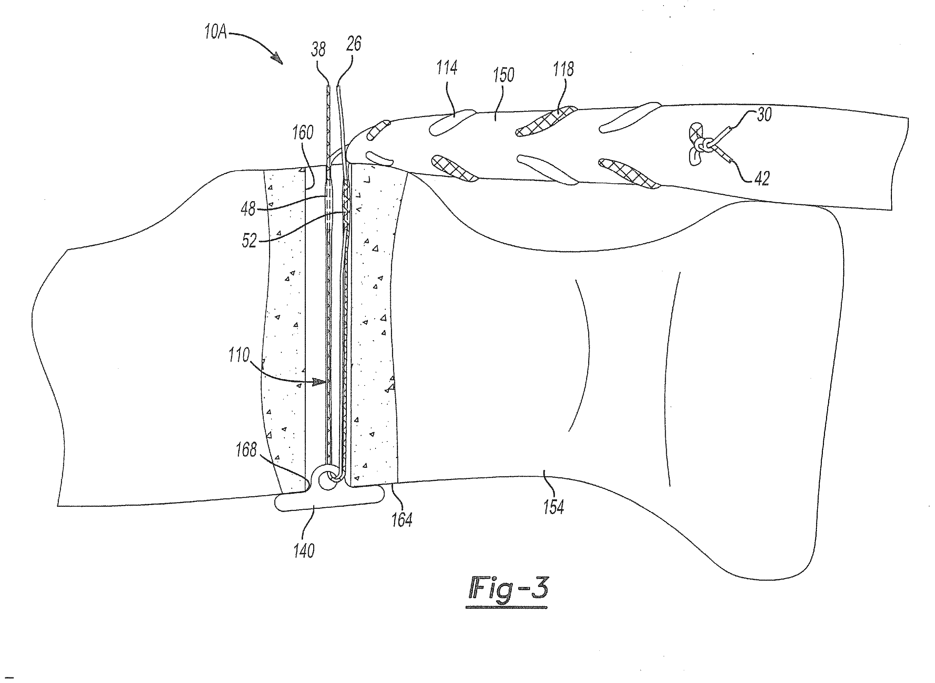

Operation of the adjustable flexible member construct 10 will now be discussed in further detail with reference to an exemplary surgical technique shown in Figure 3 where adjustable flexible member construct assembly 10A is used to attach a distal bicep tendon 150 and corresponding muscle to a radius bone 154. It should be appreciated, however, that adjustable flexible member construct 10 can be used in various attachment and/or attachment configurations, other than the example discussed above, to secure soft tissue to bone or another portion of the anatomy.

The needles 144 can be used to secure the fixed portions 114, 118 of adjustable flexible member construct 10 directly to the distal bicep tendon 150 via any suitable method, such as the whip stitch shown in Figure 3. The construction of adjustable flexible member construct 10 provides for being able to secure the second ends 30, 42 that form the ends of the fixed portions 114, 118 directly to the soft tissue without requiring an intermediate fixation member to facilitate fixation of the adjustable construct 10 to the soft tissue. Such a configuration can reduce the complexity of the procedure as well as the apparatus used to secure the soft tissue to the bone. With the fixed portions being secured to the distal bicep tendon 150, the needles can be removed, and second ends 30, 42 can be coupled together, such as with a knot, as shown in Figure 3.

The anchor member 140 can be passed through a bore 160 formed through the radius bone 154 and secured relative to an outer surface 164 of the radius bone 154 adjacent an opening 168 of bore 160. The first ends 26, 38 of flexible members 14, 18 that form adjustable construct 10 can then be tensioned to reduce a size of the adjustable portion 110 and draw the distal bicep tendon 150 into secure engagement with the radius bone 154, as shown in Figure 3. As will be discussed below, the adjustable flexible member construct 10 can maintain the reduced size of the adjustable portion 110 and corresponding tension in the adjustable flexible member construct 10 without the use of a knot, as will be discussed below.

The pulling of first ends 26, 38 can cause movement of flexible member portions 126, 130 relative to passage portions 48, 52 such that the adjustable portion 110 can be reduced to a desired size and/or placed in a desired tension. Tension in flexible member portions 126, 130 and corresponding fixed portions 114, 118 can cause the bodies 22, 34 defining passage portions 48, 52 to be placed in tension and therefore constrict about flexible member portions 102, 106 passed therethrough. This constriction reduces the diameter of passage portions 48, 52, thus forming a mechanical interface between the exterior surfaces of portions 102, 106 and an interior surface of passage portions 48, 52. This constriction results in static friction between the interior and exterior surfaces at the mechanical interface, causing the adjustable flexible members 14, 18 to "automatically" lock in the reduced size or diameter configuration in which tension is maintained without requiring a knot or other additional tying technique to maintain such tension.

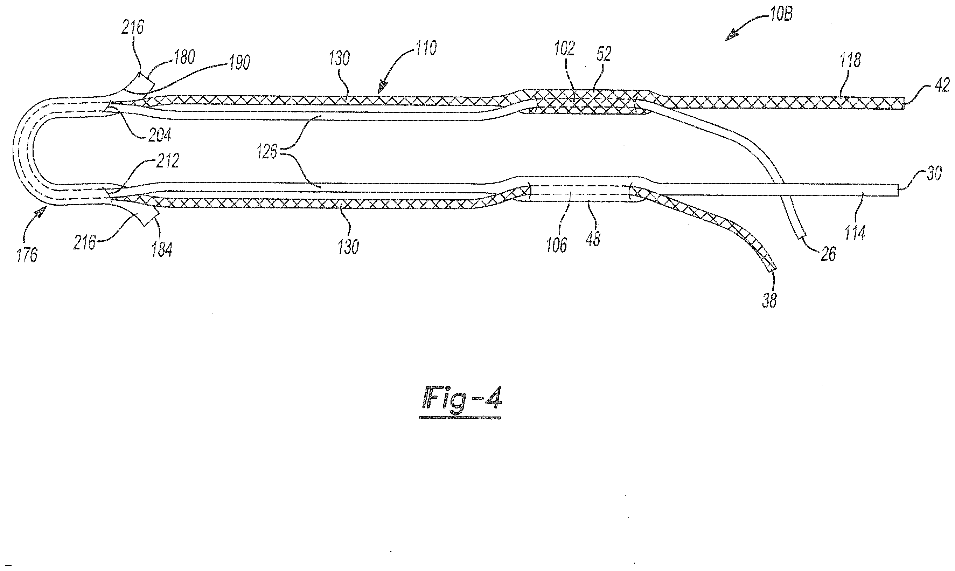

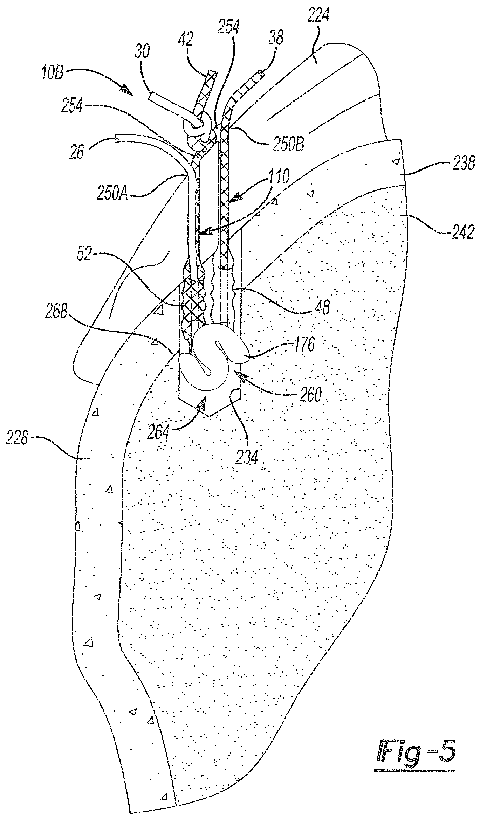

With additional reference to Figures 4 and 5, adjustable flexible member construct 10 is shown in an assembly configuration 10B where a flexible anchor 176 is coupled thereto. Flexible anchor 176 can be coupled to adjustable portion 110 in a similar position to anchor member 140, as shown in Figure 4. Flexible anchor 176 can be an elongate member having a sleeve or tubular configuration with first and second ends 180, 184 and an internal passage 190 extending therebetween. The flexible anchor 176 can be made of resorbable or non-resorbable materials, including a hollow-core braided suture, sponges and sponge-like materials in solid form, perforated materials, woven/braided from biocompatible materials or fibers, such as, for example, polymer, polyester, polyethylene, cotton, silk, or other natural or synthetic materials.

The flexible anchor 176 can have any properties that allow it to change shape. In this regard, the flexible anchor 176 can be, for example, compliant, flexible, foldable, squashable, squeezable, deformable, limp, flaccid, elastic, low-modulus, soft, spongy or perforated, or have any other characteristic property that allows it to change shape. In some aspects, the flexible anchor 176 can be coated with biological or biocompatible coatings, and also can be soaked in platelets and other biologics, which can be easily absorbed by the flexible anchor 176. In one exemplary configuration, the flexible anchor 176 can be formed from a strand of No. 5 braided polyester suture. In other words, multiple fibers can be braided together to form a hollow braided flexible member having an internal passage.

As shown for example in Figure 4, adjustable flexible member construct 10 can be passed through a first opening 204 in a wall of the flexible anchor 176, guided into and along the internal passage 190, and passed out of the internal passage 190 through a second opening 212 in a wall of the flexible anchor 176 to associate flexible anchor 176 with adjustable portion 110. The openings 204, 212 can be positioned intermediately between the first and second ends 180, 184 of the flexible anchor 176 at a distance of, for example, one-quarter length from ends 180, 184. It will be appreciated that the openings 204, 212 can be apertures or voids in the woven fabric of the flexible anchor 176, such that the openings 204, 212 do not disrupt or break the weave of the flexible anchor 176 when made of braided or woven material. Further, portions of the flexible anchor 176 between the first and second ends 180, 184 and the corresponding first and second openings 204, 212, can define anchoring leg or tail portions 216 that can provide additional resistance for securing the flexible anchor 176 relative to a bone, fastener or implant, as will be discussed in greater detail herein.

In operation, adjustable flexible member construct assembly 10B with flexible anchor 176 can operate in a similar manner as the assembly configuration 10A with anchor member 140 discussed above. In this regard, it should be appreciated that adjustable flexible member construct assembly 10B could be used in place of adjustable flexible member construct assembly 10A to secure the distal bicep tendon 150, as well as in other soft tissue securing techniques.

For example, and with reference to Figures 2 and 4-5, adjustable flexible member construct assembly 10B can be used to secure a rotator cuff 224 to a humerus bone 228. In the exemplary technique depicted in Figure 5, a bore 234 is formed in the humerus 228 through the cortical bone layer 238 and into the cancellous bone layer 242. The flexible anchor 176 can be positioned in bore 234 and the second ends 30, 42 can be passed through rotator cuff 224 via needles 144 or another suitable method at locations 250A and 250B spaced apart from each other. First end 26 can also be passed through location 250A along with second end 42 and first end 38 can be passed through second location 250B along with second end 30, as shown in Figure 5.

The second ends 30, 42 of fixed portions 114, 118 extending through rotator cuff 224 can then be tied in a knot or secured together in another suitable manner to form a loop portion 254 over rotator cuff 224, as also shown in Figure 5. Tension can then be applied to first ends 26, 38 to reduce a size of adjustable portion 110 and secure rotator cuff 224 to humerus 228. In applying tension to first ends 26, 38, flexible anchor 176 can be drawn into engagement with cortical bone layer 238 to set the flexible anchor 176 in an anchoring configuration or mass relative to cortical bone layer 238, as also shown in Figure 5. In one exemplary configuration, during setting of flexible anchor 176, portions of the anchor, including tail portions 216, can bunch together, collapse, expand and/or change shape to a second shape, configuration or locking profile 260 to form an anchoring mass 264.

Anchoring mass 264 can then be set or seated against an inner face of cortical bone layer 238 surrounding bore 234. In an exemplary configuration, second shape or profile 260 can include a width that is greater than that of the initially formed bore 234 such that portions of flexible anchor 176 can expand into the cancellous bone layer 242 and extend transversely beyond the width or diameter of bore 234 beneath the cortical bone 238. For example, the anchoring mass 264 can include a width in a direction perpendicular to a longitudinal axis of bore 234 greater than the width of initially formed bore 234. In one exemplary configuration, the flexible anchor 176 can lock against a ledge 268 of cortical bone layer 238, as also shown in Figure 5.

Upon seating of the flexible anchor 176, or in combination therewith, tension applied to first ends 26, 38 can draw loop portion 254 against rotator cuff 224 and thus draw rotator cuff 224 in secure engagement with humerus 228. As with the other techniques discussed above, adjustable flexible member construct assembly 10B can automatically lock under tension and/or load without requiring an additional knot to maintain the tension.

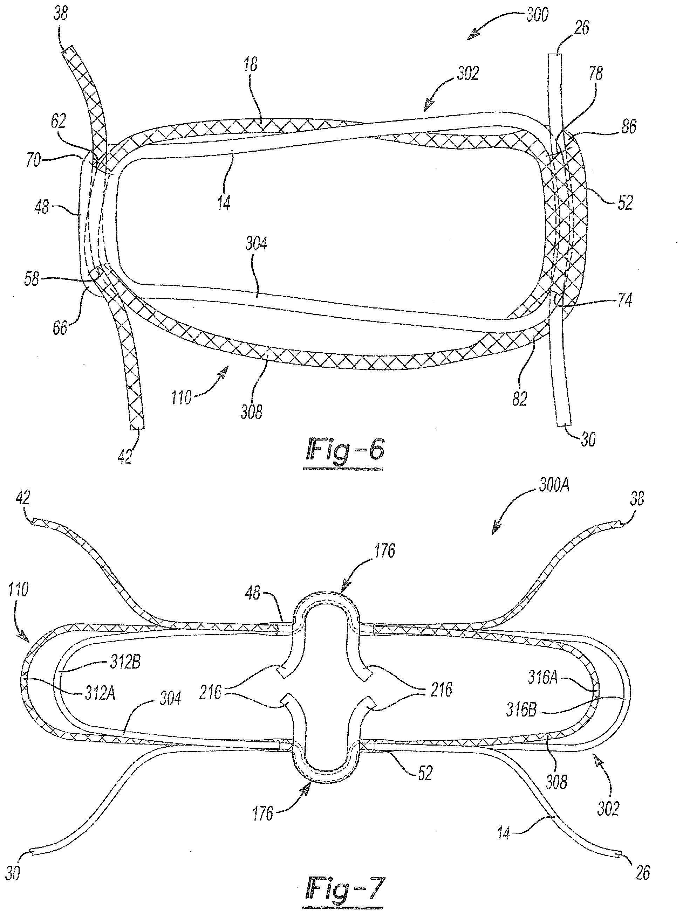

Turning now to Figures 6-8, an adjustable flexible member construct 300 is provided in accordance with the present teachings. With particular reference to Figure 6, adjustable flexible member construct 300 can include a double loop configuration and can be optionally formed using the adjustable flexible member construct 10 discussed above. As can be seen in Figure 6, adjustable flexible member construct 300 can include first and second ends 26, 30 of flexible member 14 extending from opposite ends of passage portion 52 of flexible member 18, and first and second ends 38, 42 of flexible member 18 extending from opposite ends of passage portion 48 of flexible member 14.

To form adjustable flexible member construct 300, second end 42 of flexible member 18 of adjustable construct 10 can be passed into passage portion 48 via second aperture 62 and out passage portion 48 via first aperture 58. Similarly, second end 30 of flexible member 14 of adjustable construct 10 can be passed into passage portion 52 via fourth aperture 78 and out passage portion 52 via third aperture 74 to form the cooperating double self-locking adjustable loop configuration shown in Figure 6. In the exemplary configuration illustrated, the first and second ends 26, 30 of flexible member 14 pass through passage portion 52 in opposite directions and the first and second ends 38, 42 of flexible member 18 pass through passage portion 48 in opposite directions. Thus, the first and second ends 26, 30 extend from respective opposite ends 86, 82 of passage portion 52 and the first and second ends 38, 42 extend from respective opposite ends 70, 66 of passage portion 48. This configuration can thus form a first adjustable loop 304 from flexible member 14 in cooperation with passage portion 52 of flexible member 18, and a second adjustable loop 308 from flexible member 18 in cooperation with passage portion 48 of flexible member 14. In other words, two adjustable portions are formed between passage portions 48, 52, namely the adjustable portion 110 and another adjustable portion 302. In the exemplary configuration illustrated, when adjustable flexible member construct 300 is placed under tension, the first adjustable loop 304 can self-lock in cooperation with passage portion 52 and the second adjustable loop 308 can self-lock in cooperation with passage portion 48. In one exemplary aspect, the first and second adjustable loops 304, 308 can be co-locking adjustable loops of self-locking adjustable flexible member construct 300.

Figure 7 illustrates adjustable flexible member construct 300 in an assembly configuration 300A where a pair of flexible anchors 176 are coupled to the respective passage portions 48, 52. It should be appreciated, however, that the pair of flexible anchors 176 could alternatively be different fixation members and/or could be coupled to first portions 312A, 312B of both the first and second loops 304, 308 and second portions 316A, 316B of both the loops 304, 308 of adjustable flexible member construct 300.

The longitudinal and parallel placement of the first and second ends 26, 30 of flexible member 14 within and through passage portion 52 and the first and second ends 38, 42 of flexible member 18 within and through passage portion 48 resists the reverse relative movement of the first and second ends of each of flexible members 14, 18 once flexible member construct 300/300A is tightened. Upon applying tension to the first and second ends 26, 30 and the first and second ends 38, 42, adjustable portions 110, 302 can be reduced to a desired size or placed in a desired tension. Tension in the adjustable portions 110, 302 can cause the bodies of the flexible members 14, 18 defining the passage portions 48, 52 to be placed in tension and therefore constrict about the portions of flexible members 14, 18 extending therethrough similarly to the constriction discussed above with respect to adjustable flexible member construct 10. This constriction can cause the adjustable flexible member construct 300/300A to "automatically" lock in a reduced size or smaller diameter configuration and maintain the tension without requiring a knot.

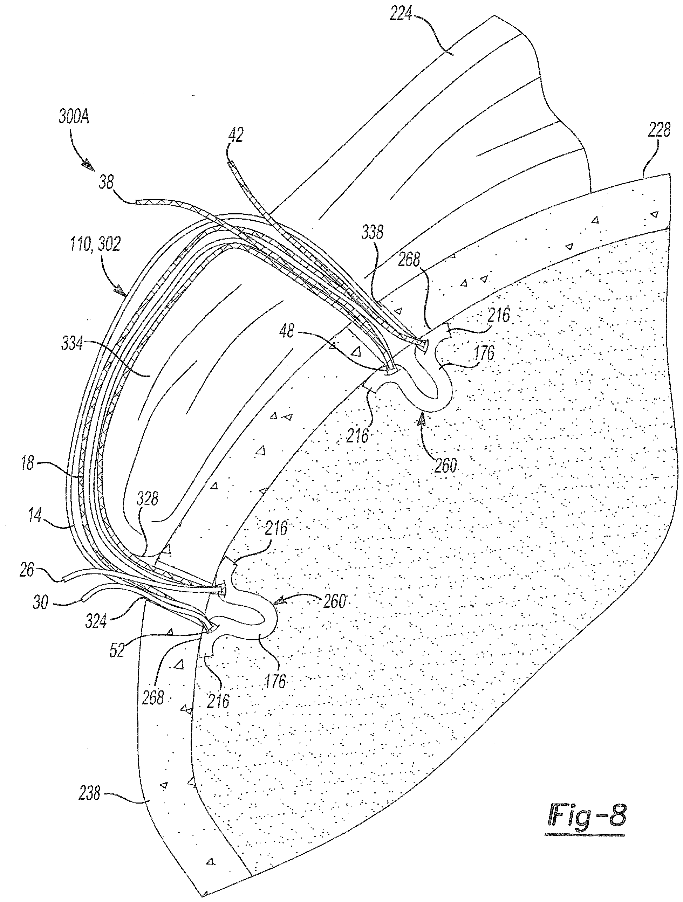

With particular reference to Figure 8, an exemplary technique for coupling soft tissue to bone with adjustable flexible member construct assembly 300A will now be discussed in accordance with the present teachings. In one exemplary aspect, adjustable flexible member construct assembly 300A can be used to secure the rotator cuff 224 to the humerus 228. In this aspect, the flexible anchor 176 coupled to passage portion 52 can be positioned in a first bore 324 formed in the humerus 228 in a similar manner as bore 234 discussed above. In the exemplary aspect illustrated, first bore 324 can be formed in humerus 228 adjacent an end 328 of rotator cuff 224, as shown in Figure 8. The adjustable construct assembly 300A can then be positioned over a portion 334 of the rotator cuff 224 and pierced through rotator cuff 224 such that the flexible anchor 176 coupled to the second passage portion 48 is positioned within a second bore 338 spaced apart from the first bore 324. At this point, the adjustable portions 110, 302 can extend from each of the passage portions 48, 52 over the rotator cuff 224, as shown in Figure 8.

Tension can then be applied to the first and second ends 26, 30 of flexible member 14 and the first and second ends 38, 42 of flexible member 18 to reduce a size of the adjustable portions 110, 302 and draw the rotator cuff 224 into secure engagement with the humerus 228. As discussed above, tensioning the first and second ends 26, 30, 38, 42 places the adjustable portions 110, 302 under tension thereby causing the passage portions 48, 52 to constrict and automatically lock the flexible members 14, 18 in place under the desired tension without the use of a knot. Further, tensioning the free ends 26, 30, 38, 42 can draw flexible anchors 176 in bores 324, 338 against the cortical bone layer 238 such that tail portions 216 engage the ledge 268 of cortical bone layer 238 thus changing a shape of the flexible anchors 176 from a first profile when the flexible anchors 176 are inserted into the bores 324, 338 to the second profile 260 shown in Figure 8 where tail portions engage the ledge 268 of cortical bone layer 238. In one exemplary configuration, flexible anchors 176 can change from the first shape or profile to the second shape or profile 260 forming anchoring mass 264 against ledge 268, as shown in Figure 8 with reference to Figure 5.

It should be appreciated that while the rotator cuff technique has been discussed above in connection with placing passage portion 48 in bore 338 and passage portion 52 in bore 324, either passage portion 48, 52 could be placed in either bore 324, 338. Further, flexible anchors 176 coupled to first portions 312A, 312B and second portions 316A, 316B could alternatively be positioned in the bores 324, 338. In addition, more than one adjustable flexible member construct assembly 300A could be utilized to secure the rotator cuff 224 to humerus 228 using the same or additional bores formed in humerus 228.

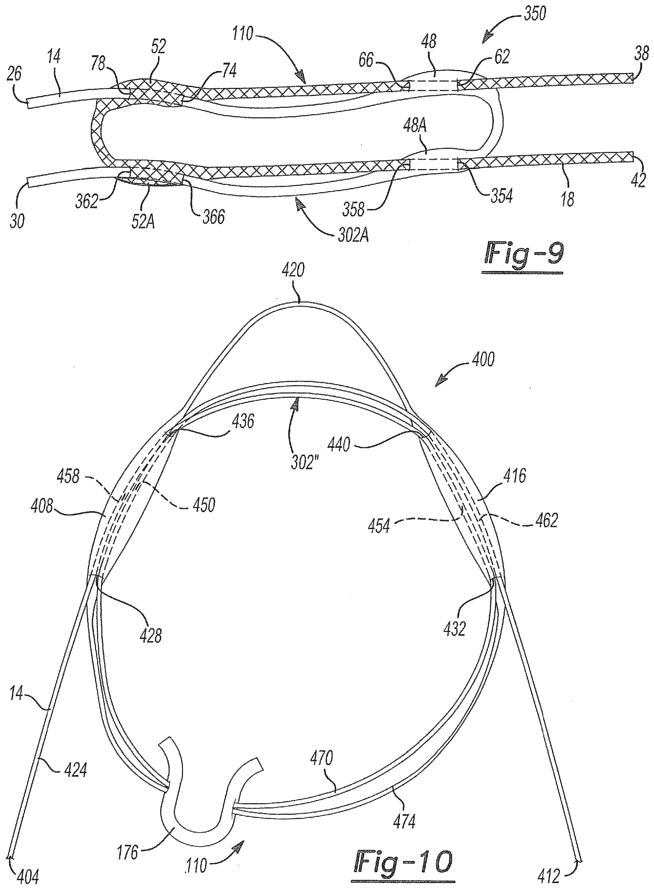

Turning now to Figure 9, an adjustable flexible member construct 350 is provided according to the present teachings. The adjustable flexible member construct 350 can include a double loop configuration as well as two passage portions 48, 48A defined by flexible member 14 and two passage portions 52, 52A defined by flexible member 18. Adjustable flexible member construct 350 can also be formed based on the adjustable flexible member construct 10 discussed above. In this regard, flexible member 14 can include the second passage portion 48A and flexible member 18 can include the second passage portion 52A. In the exemplary configuration illustrated, each of the second passage portions 48A, 52A are spaced apart from the corresponding first passage portions 48, 52. Passage portion 48A can include a fifth aperture 354 and a sixth aperture 358, and passage portion 52A can include a seventh aperture 362 and an eighth aperture 366.

Using adjustable flexible member construct 10 with the second passage portions 48A, 52A discussed above, second end 30 of flexible member 14 can be passed into passage portion 52A via aperture 366 and out via aperture 362, as shown in Figure 9. Similarly, second end 42 of flexible member 18 can be passed into passage portion 48A via aperture 358 and out via aperture 354. This construction can provide a double loop configuration similar to construct 300, but with four passage portions. In this regard, adjustable portion 110 can remain between passage portions 48 and 52, as shown in Figure 9. However, adjustable portion 302, depicted in Figure 9 as 302A, can be positioned between second passage portions 48A and 52A. Adjustable flexible member construct 350 can automatically lock when placed under tension similar to the constructs discussed above, and can also be provided in various assembly configurations, such as with flexible anchors 176. In this regard, construct 350 can be used to secure soft tissue to bone, such as in the exemplary rotator cuff technique discussed above, as well as to compress two bone portions together, such as discussed in commonly owned, co-pending U.S. Pub. Nos. 2010/0211075 and 2011/01061 53.

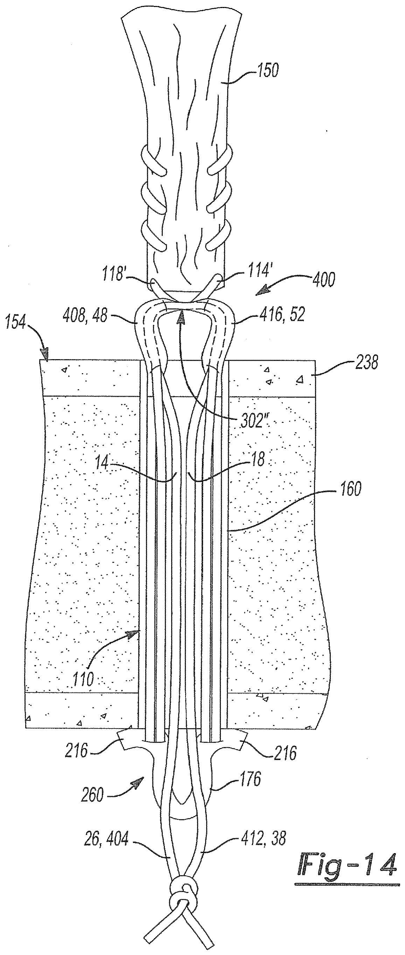

With additional reference to Figures 10-14, an adjustable flexible member construct 400 is provided in accordance with the present teachings. Figures 10-13 illustrate an exemplary method of forming construct 400 and Figure 14 illustrates an exemplary technique of securing soft tissue to bone using construct 400. The adjustable flexible member construct 400 can be fashioned from either a single flexible member, such as flexible member 14, or from two flexible members, such as by using adjustable flexible member construct 10 as a starting point.

Forming adjustable flexible member construct 400 from a single flexible member, such as flexible member 14, will now be discussed with particular reference to Figures 10-13. In this aspect, the adjustable flexible member construct 400 can include a first end 404, a first formed passage portion 408, a second end 412, a second formed passage portion 416, and a fixed length loop portion 420 (when formed from a single flexible member) connecting the first and second passage portions 408, 416, as shown in Figure 10. In the exemplary configuration illustrated, flexible member construct 400 can include an elongated body 424 having an exterior surface and an interior surface defining an elongated passage between the first and second ends 404, 412. The body 424 can define the first and second passage portions 408, 416 and the fixed length portion 420 therebetween. Passage portions 408, 416 can each include first apertures 428, 432 positioned proximate one end thereof, and second apertures 436, 440 positioned proximate a second opposite end thereof. The passage portions 408, 416 can be formed to have a larger width or diameter than remaining portions of flexible member 14, as also shown in Figure 10. Alternatively, the passage portions 408, 416 can be formed initially to have the same width or diameter as the remaining portions of flexible member 14, later expanding in diameter during the construction process. In various aspects, the first and second apertures 428, 432, 436, 440 can be formed during a braiding process of flexible member 14 as loose portions between pairs of fibers defining flexible member 14, or can be formed during the construction process. Alternatively, the first and second ends can be pushed between individual fibers of the braided flexible member 14.

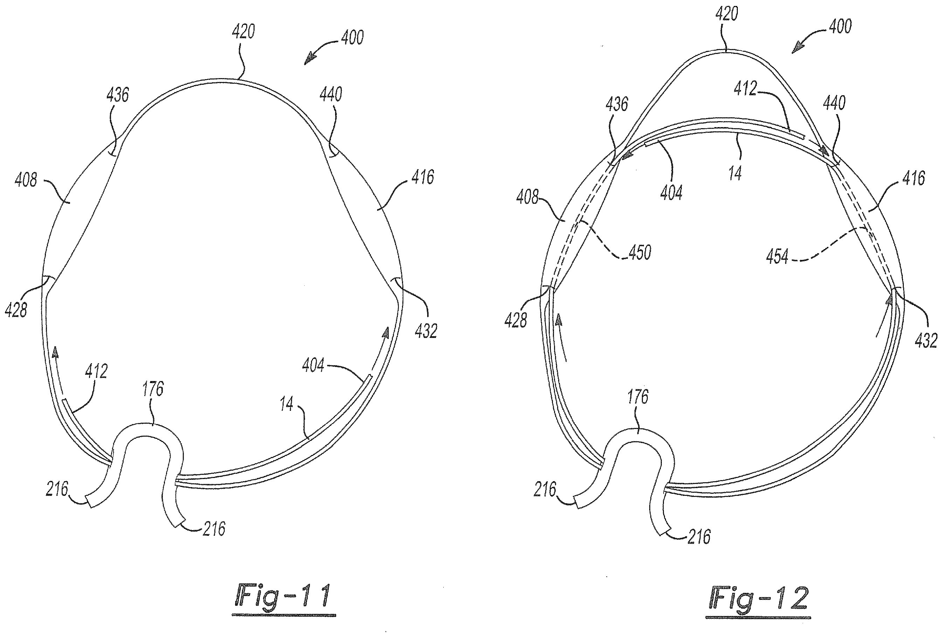

The first end 404 can be passed through second passage portion 416 via first and second apertures 432, 440, as generally shown in Figures 11 and 12. In a similar manner, second end 412 can be passed through the first passage portion 408 via the first and second apertures 428, 436, as also shown in Figures 11 and 12. Subsequently, as shown in Figure 12 with reference to Figure 10, first end 404 can be passed through the first passage portion 408 via second and first apertures 436, 428, respectively. First end 404 can follow a path that is opposite in direction to a path followed by a portion 450 of the flexible member 14 that has already passed through first passage portion 408 while following second end 412 through first and second apertures 428, 436.

Similarly, second end 412 can be passed through the second passage portion 416 via second and first apertures 440, 432, respectively. Second end 412 can follow a path that is opposite in direction to a path followed by a portion 454 of the flexible member 14 that has already passed through second passage portion 416 while following first end 404 through first and second apertures 432, 440. This results in portions 458, 462 of flexible member 14 being positioned parallel or substantially parallel to portions 450, 454 in passage portions 408, 416. Passing the first and second ends 404, 412 through passage portions 408, 416 as discussed above forms adjustable loops 470, 474, as shown in Figure 10. The first and second ends 404, 412 can be passed through the same apertures in each passage portion 408, 416 or, alternatively, through separate apertures in each passage portion 408, 416.

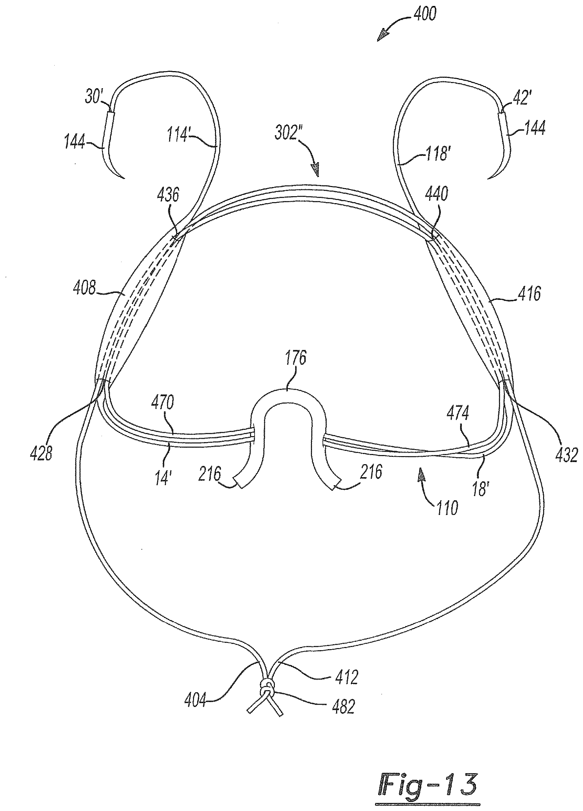

The fixed portion 420 can then be cut, as shown in Figure 13, to effectively form two flexible members 14' and 18' having fixed length portions 114', 118' with ends 30' and 42'. Needles 144 or other flexible member passing instruments can be coupled to ends 30', 42' and a flexible anchor 176 can be coupled to loops 470, 474 to form the construct 400, as illustrated in Figure 13. Ends 404, 412 can also be tied in an optional knot 482.

The adjustable flexible member construct 400 can thus provide a double adjustable loop configuration via loops 470, 474 while also providing fixed portions 114', 118' extending from passage portions 408, 416. As will be discussed in greater detail herein, this configuration can be used, for example, to couple soft anchor 176 to loops 470, 474 and couple fixed length portions 114', 118' directly to soft tissue.

In another exemplary aspect, adjustable flexible member construct 400 can be formed starting with two separate flexible members, such as flexible members 14, 18. For example, and with reference to Figure 13 and adjustable flexible member construct assembly 10A discussed above in Figure 4, first end 26 of flexible member 14 can be passed into passage portion 48 via second aperture 62 and out via first aperture 58 thereby passing through passage portion 48 in an opposite direction as flexible member 18. In a similar manner, first end 38 of flexible member 18 can be passed into passage portion 52 via fourth aperture 78 and out via third aperture 74 thereby passing through passage portion 52 in an opposite direction as flexible member 14. This technique can thus also be used to form adjustable flexible member construct 400 having fixed length portions 114', 118' and two adjustable loops 470, 474 formed by adjustable portions 110 and 302" extending between passage portions 408, 416.

With particular reference to Figures 13 and 14, operation of adjustable flexible member construct 400 will now be discussed in greater detail in connection with an exemplary technique where construct 400 is used to attach soft tissue to bone. In one exemplary aspect, fixed portions 114', 118' can be coupled to soft tissue, such as the distal bicep tendon 150, using needles 144. In one exemplary configuration, fixed portions can be 114', 118' can be directly sutured to the soft issue, such as via the whip stitch shown in Figure 14, and then the remaining fixed portions 114', 118' and needle 144 can be removed. The ends 404, 412 can be optionally tied together and passed through the bore 160 formed in the radius bone 154 along with the a portion of the loops 470, 474 such that flexible anchor 176 is positioned through bore 160.

Tension can then be applied to ends 404, 412 to reduce a size of loops 470, 474 and/or adjustment portions 110, 302" and draw the distal bicep tendon 150 toward radius bone 154 and into secure engagement therewith. Tensioning ends 404, 412 can place the bicep tendon 150 and associated muscle, as well as the flexible members 14', 18' of the adjustable construct 400 under a desired tension. Similar to the constructs discussed above, tension in flexible members 14, 18 can cause the passage portions 408, 416 to constrict and thereby automatically lock the adjustment portions 110, 302" to maintain the desired tension without the use of a knot.

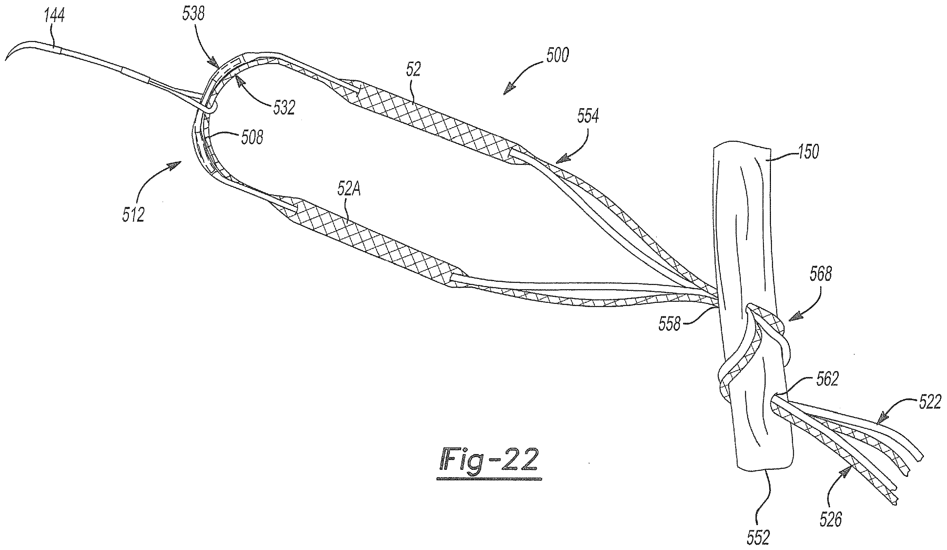

With additional reference to Figures 15-29, an adjustable flexible member construct 500 and associated exemplary surgical technique will now be discussed in accordance with various aspects of the present teachings. As will be discussed in greater detail below, adjustable flexible member construct 500 can be formed from two separate flexible members and can facilitate coupling a fixed portion of the construct directly to soft tissue without requiring an intermediate coupling member.

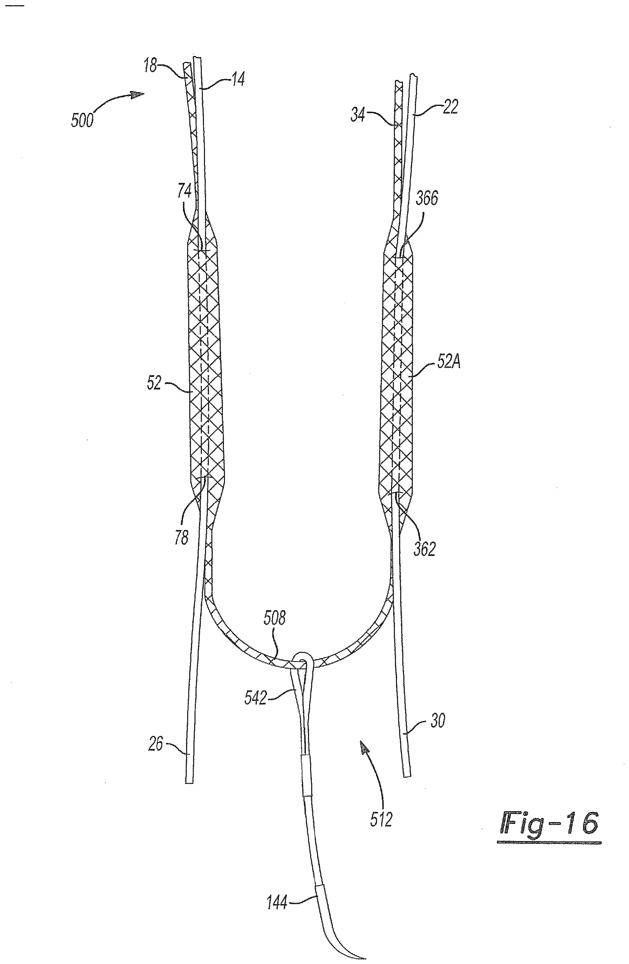

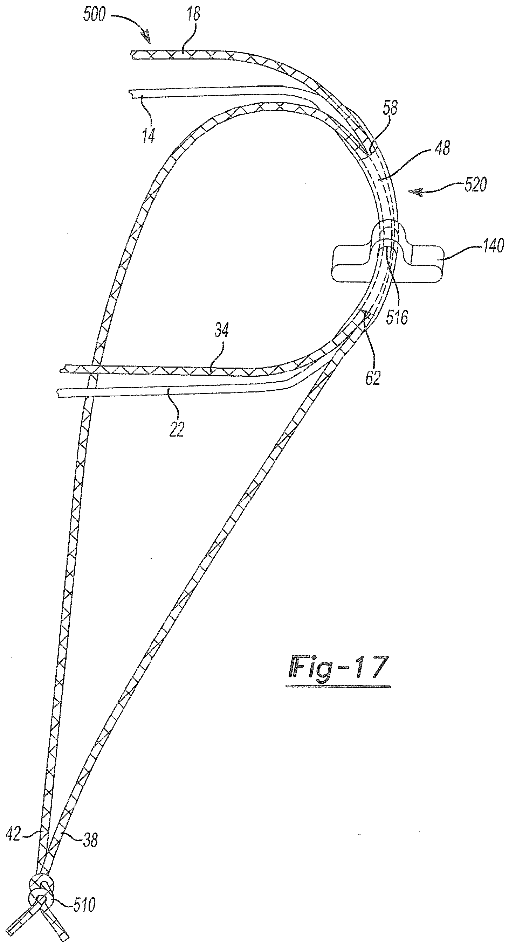

The adjustable flexible member construct 500 can be fashioned from the first and second flexible members 14, 18 and, as will become apparent from the discussion below, can include features similar to aspects of adjustable flexible member construct 300 shown in Figure 6 and adjustable flexible member construct 350 shown in Figure 9. In this configuration, the body 22 of first flexible member 14 can define one passage portion 48 having first and second apertures 58, 62, as generally shown in Figure 15 and the enlarged view of Figure 17. Such a configuration of flexible member 14 is also shown in Figure 6, with particular reference to a left hand side of construct 300. The body 34 of flexible member 18 can define two passage portions 52, 52A having respective apertures 74, 78 and 362, 366, as generally shown in Figure 15 with reference to the enlarged view of Figure 16. As can be seen, passage portions 52, 52A can be spaced apart from each other by a fixed portion 508 of flexible member 18. Such a configuration of flexible member 18 is also shown in Figure 9, with particular reference to a left hand side of construct 350.

To form adjustable flexible member construct 500, the first end 26 of flexible member 14 can be passed into and through passage portion 52 via apertures 74, 78 and second end 30 can be passed into and through passage portion 52A via apertures 366, 362. The first end 38 of flexible member 18 can be passed into and through passage portion 48 via apertures 58, 62, and the second end 42 can be passed through passage portion 48 in an opposite direction as first end 38 via apertures 62, 58. It should be appreciated that first and second ends 38, 42 can be passed through passage portion 48 via the same or different apertures.

First and second ends 38, 42 can then optionally be tied in a knot 510 or otherwise coupled together, as shown in Figure 15. In the exemplary configuration illustrated in Figures 15-29, adjustable flexible member construct 500 can include needle 144 slidably coupled to the fixed portion 508 at a first end 512 of construct 500, and anchor member 140 coupled to passage portion 48 via aperture 516 at a second opposite end 520 of construct 500. In this exemplary configuration, passage portion 48, as well as first and second ends 38, 42 of flexible member 18 extending therethrough are slidably positioned through aperture 516 of anchor member 140, as shown in Figure 17. While adjustable flexible member construct 500 is shown and discussed in connection with needle 144 and anchor member 140, it should be appreciated that construct 500 can be used with or without needle 144 and anchor member 140 and/or with other suitable suture passing members and/or fixation members, such as flexible anchor 176.

This configuration of adjustable flexible member construct 500 can form first and second adjustment portions 522, 526 extending between passage portions 52, 52A of flexible member 18 and passage portion 48 of flexible member 14, as shown in Figure 15. Further, the looped configuration of flexible member 18 in cooperation with passage portions 48, 52, 52A can also provide an adjustable loop 532. As will be discussed in greater detail below, tension can be applied to ends 26, 30 of flexible member 14 and ends 38, 42 of flexible member 18 to reduce a size of loop 532 and adjustment portions 522, 526. In this regard, adjustable flexible member construct 500 can be used to attach soft tissue to bone and automatically lock the suture construct 500 via passage portions 48, 52 and 52A at a desired size or tensile load without the use of a knot.

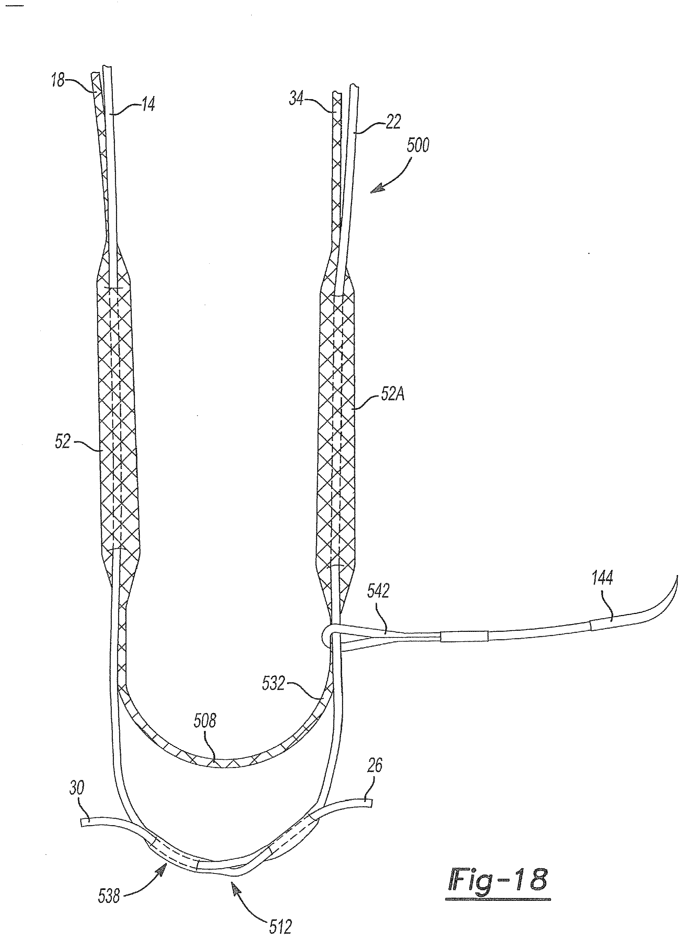

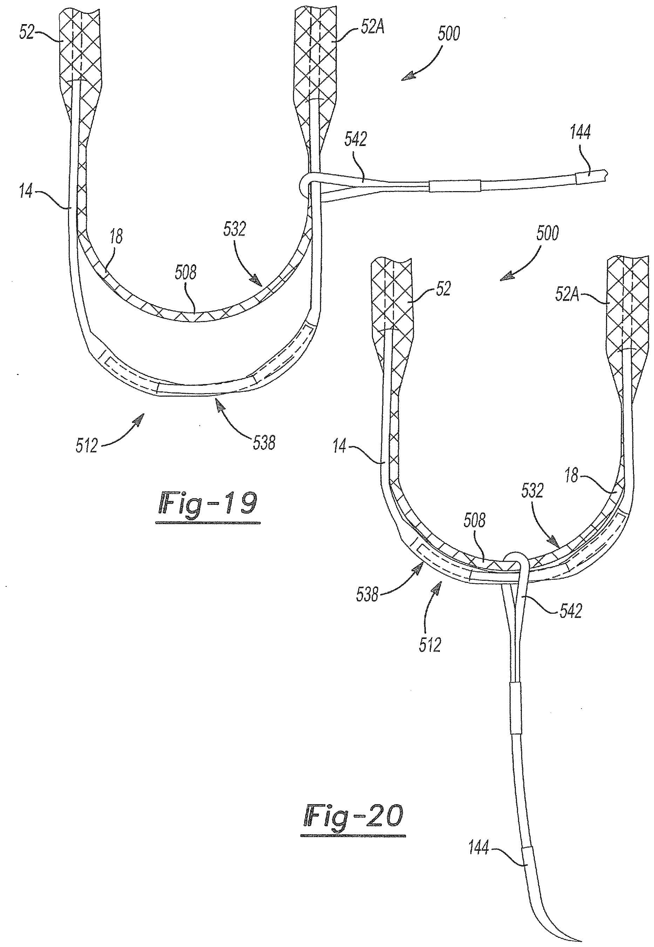

To facilitate coupling the first end 512 of adjustable flexible member construct 500 to soft tissue, free ends 26, 30 at the first end 512 can be optionally passed or tucked inside body 22 and can form a loop portion 538, as shown in Figure 18. Any remaining portions of ends 26, 30 extending from body 22 can then be trimmed, as shown in Figure 19. Before tucking the free ends 26, 30 into body 22, one of the free end 26 or 30 can be passed inside the coupling loop 542 such that needle 144 can then be passed around both fixed portion 508 and formed loop portion 538, as also shown in Figure 19. A size of loop 532 can be optionally adjusted relative to passage portion 48 to substantially align fixed portion 508 with formed loop portion 538, as shown in Figure 20. Having the fixed portion 508 align with formed loop portion 538 can facilitate easier passing of construct 500 through soft tissue, as will be discussed below.

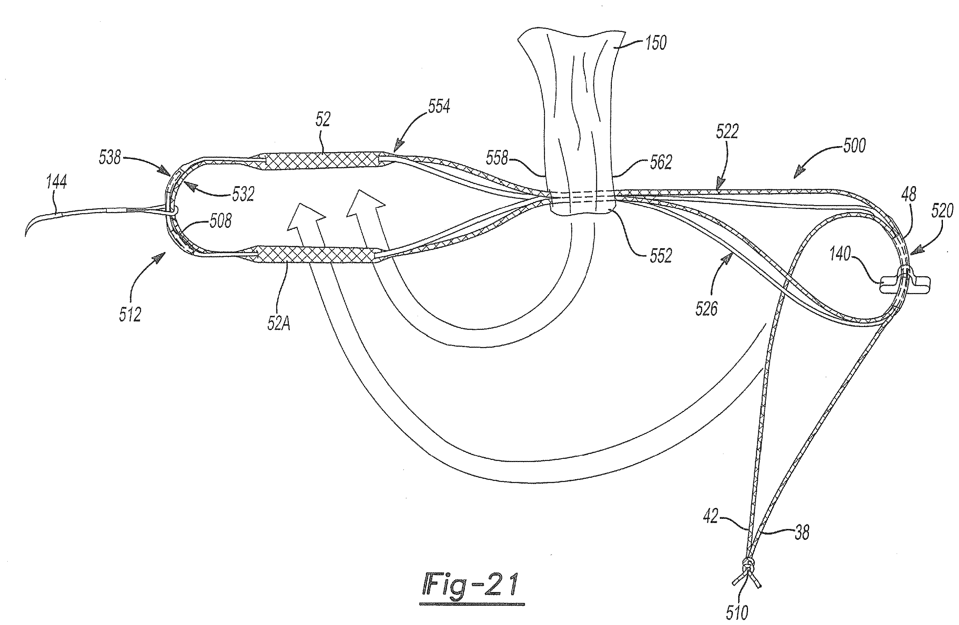

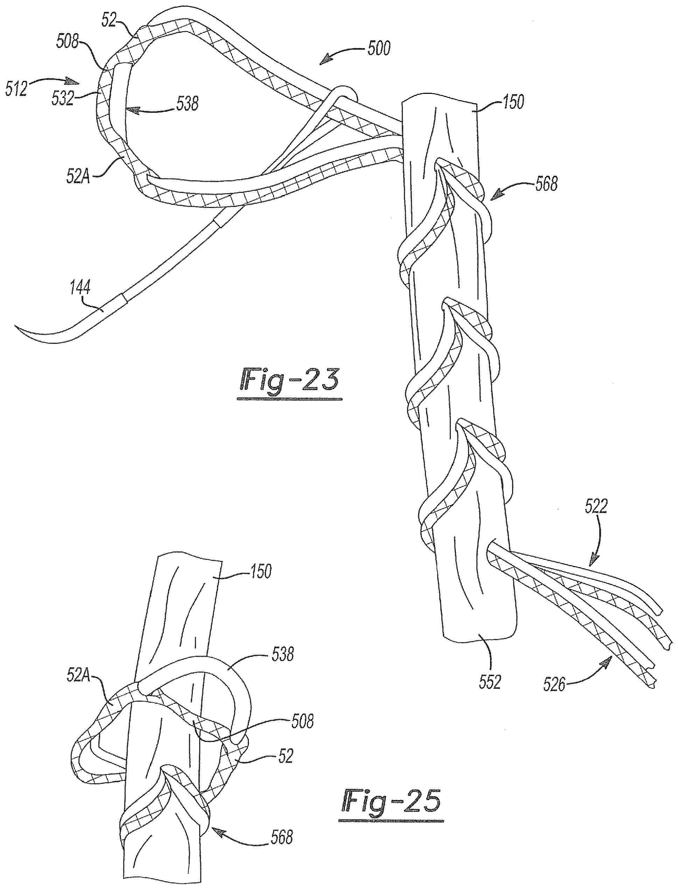

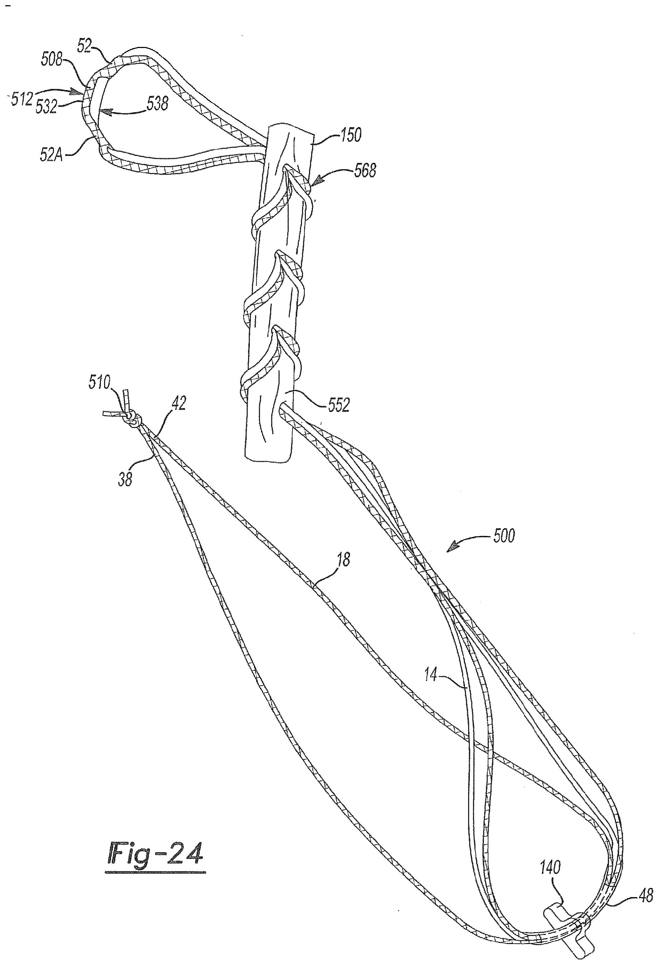

With particular reference to Figures 21-24, coupling adjustable flexible member construct 500 to soft tissue will now be discussed in greater detail. In the exemplary configuration illustrated, construct 500 is shown being coupled to distal bicep tendon 150. It should be appreciated, however, that construct 500 can be used in various techniques for coupling soft tissue to bone, such as in an ACL construction procedure, for example. Needle 144 can be used to pass first end 512 of construct 500 through distal bicep tendon 150 a first time, as shown in Figure 21. The second end 520 of construct 500 along with an end 552 of distal bicep tendon 150 can be passed through a portion 554 of construct 500 extending between the first end 512 and an exit side 558 of distal bicep tendon 150 opposite an entrance side 562. Needle 144 along with first end 512 can then be passed through the entrance side 562 again and the process repeated to form a weave-like pattern 568 shown in Figure 23. The needle 144 can then be removed from first end 512, as shown in Figure 24. The pattern 568 can form a non-tortuous path for the suture so as to facilitate initial sliding of the distal bicep tendon 150 relative to flexible member 14, as will be discussed below.

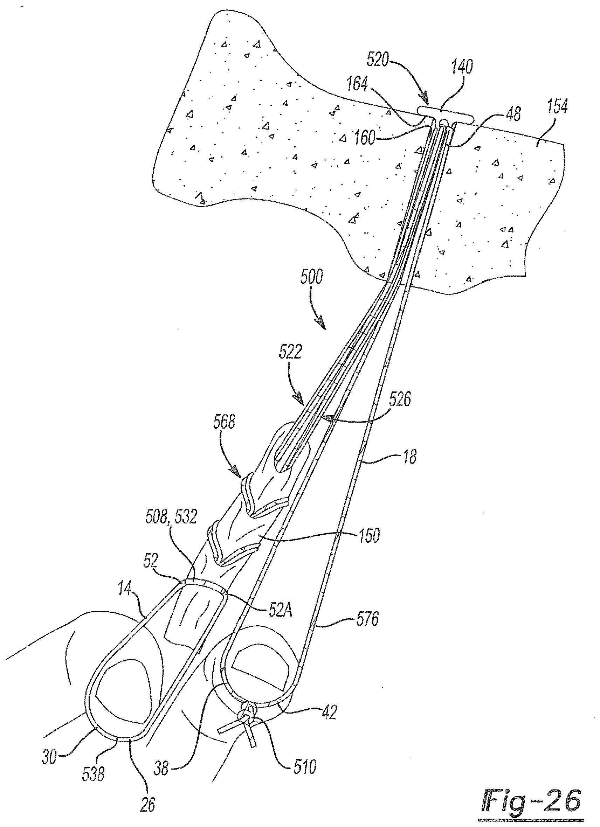

With additional reference to Figures 25-29, attaching the distal bicep tendon 150 to the radius bone 154 will now be discussed in greater detail. With particular reference to Figures 25 and 26, the second end 520 of construct 500 can be passed through bore 160 in radius bone 154. The formed loop portion 538 can be separated from alignment with the fixed portion 508, as shown in Figure 25, and tension can be applied to the construct 500 via ends 26, 30 and ends 38, 42. In the exemplary configuration illustrated in Figure 26, ends 26, 30 (Figure 20) are in the form of optional loop 538 and ends 38, 42 are in the form of an optional loop 576 via knot 510. In this configuration, the loops 538 and 576 can be tensioned either by hand or with any appropriate instrument. Applying tension to construct 500 in this manner can seat anchor member 140 against outer surface 164 of radius bone 154 and facilitate drawing distal bicep tendon 150 toward and into secure engagement with radius bone 154, as will be discussed below.

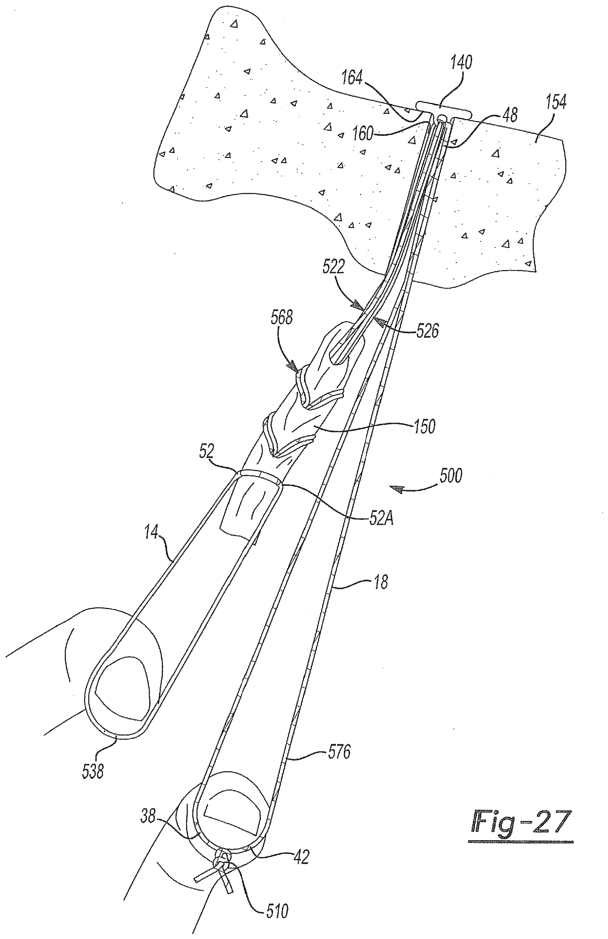

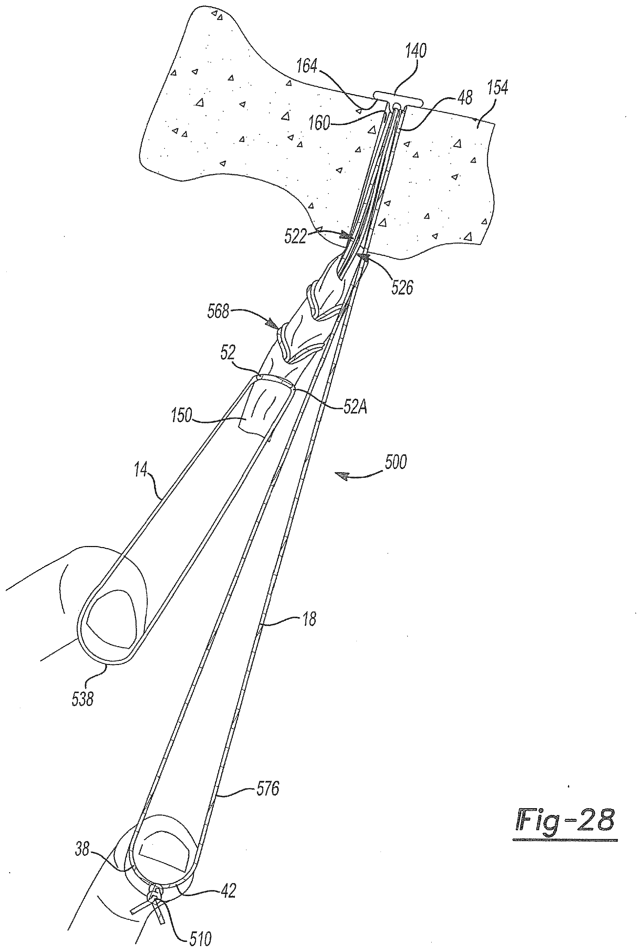

In particular, once anchor member 140 is seated against radius bone 154 with an initial amount of tension being applied to construct 500 to remove any slack from the construct, further tensioning of the flexible member 18 via loop 576 can draw fixed portion 508 against the distal bicep tendon 150 by moving the flexible member 18 relative to the flexible member 14 and the distal bicep tendon 150. In other words, flexible member 14, under tension, remains taut relative to anchor member 140 such that flexible member 14 can initially serve a function of a guidewire or guiding path for moving distal bicep tendon 150 toward radius bone 154. Further tension applied to loop 576 of flexible member 18 can shorten a length of loop 532 relative to passage portion 48 and thus draw distal bicep tendon 150 along flexible member 14 toward radius bone 154, as shown in Figures 27 and 28.

The adjustable flexible member construct 500 can provide support for holding the distal bicep tendon 150 and associated muscle to the radius bone 154 via the four flexible member strands extending between the passage portion 48 and the passage portions 52, 52A. In particular, although the flexible member 14 serves the initial function of a guidewire while drawing the distal bicep tendon 150 toward the radius bone 154, it also serves to hold the distal bicep tendon 150 at the desire tension/load via the portions of flexible member 18 that form the adjustment portions that extend between passage portion 48 at the anchor member 140 and passage portions 52, 52A at the distal bicep tendon 150. Similarly, the portions of flexible member 18 that form part of adjustment portions 522, 526 extending between the passage portions 52, 52A and passage portion 48 can also hold the bicep tendon 150 at the desired tension. In a similar manner to the constructs discussed above, the passage portions 48, 52, 52A can automatically lock the respective flexible members 18, 14 under the desired tension/load without the use of a knot.

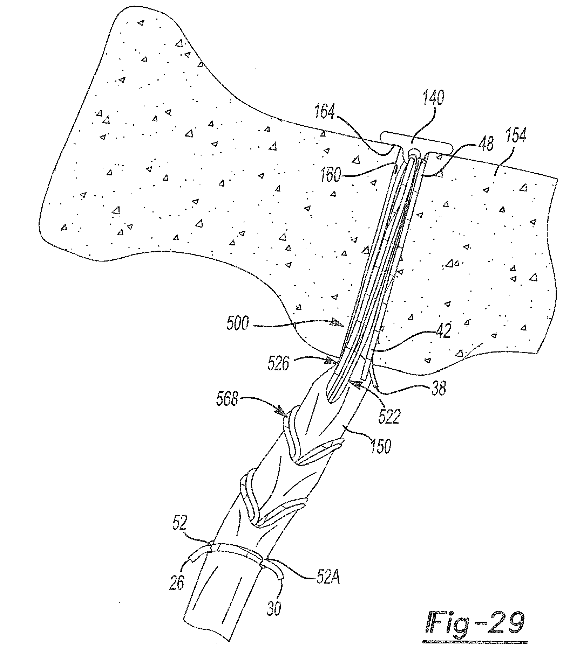

Upon tensioning flexible member 18 via loop 576 a sufficient amount to draw distal bicep tendon 150 into secure engagement with radius bone 154 under a desired tensile load, such as shown in Figure 29, the ends 26, 30 of flexible member 14 extending from passage portions 52, 52A can be trimmed. The ends 38, 42 extending from passage portion 48 and bore 160 in radius bone 154 can also be trimmed.

## Claims

1. An apparatus for use in surgical implantation, comprising:
a first flexible member (14) having a first end (26), a second end (30) and a first body (22) extending therebetween, the first body (22) defining a first passage portion (48);
a second flexible member (18) having a first end (38), a second end (42) and a second body (34) extending therebetween, the second body (34) defining second and third passage portions (52, 52a),
the first end (26) of the first flexible member (14) passing into and through the second passage portion (52) such that the first end (26) extends outside of the second passage portion (52), and the second end (30) of the first flexible member (14) passing into and through the third passage portion (52A) such that the second end extends outside of the third passage (52A) portion; and
the first and second ends (38, 42) of the second flexible member passing into and through the first passage portion (48) of the first flexible member (14) to form a self-locking adjustable flexible member construct, the apparatus further comprising the second and third passage portions (52, 52A) spaced apart from each other by a fixed length portion (508) of the body of the second flexible member (18).

2. The apparatus of claim 1, wherein the first and second ends (38, 42) of the second flexible member (18) extend into and through the first passage portion (48) in opposite directions such that the first end (38) of the second flexible member (18) extends from one end of the first passage portion (48) and the second end (42) of the second flexible member (18) extends from the other opposite end of the first passage portion (48).

3. The apparatus of claim 1, wherein the fixed length portion (508) of the second flexible member (18) in cooperation with the first and second ends (38, 42) of the second flexible member (18) passing through the first passage portion (48) forms a self- locking adjustable loop (500).

4. The apparatus of claim 1, further comprising a first adjustable length portion (522) formed by first portions of the first and second flexible members (14, 18) extending between the first and second passage portions (48, 52), and a second adjustable length portion (526) formed by separate second portions of the first and second flexible members (14, 18) extending between the first and third passage portions (48, 52A);
wherein applying tension to the first and second ends of the first and second flexible members (26, 30, 38, 42) reduces a size of the first and second adjustable length portions (522, 526).

5. The apparatus of claim 1, wherein the adjustable flexible member construct (580) is configured to automatically lock under tension to maintain the reduced size of the first and second adjustable length portions (522, 526) under tension in an absence of a knot.

6. The apparatus of claim 4, wherein the first body defines a hollow interior, wherein the first end (26) of the first flexible member passes into the hollow interior of the first body proximate the second end (30) of the first flexible member, and wherein the second end (30) of the first flexible member passes into the hollow interior of the first body proximate the first end (26) of the first flexible member (14) to form a loop portion (532) extending from an opposite side (512) of the second and third passage portions (52, 32A) as the first and second adjustable length portions (522, 526).

7. The apparatus of claim 6, further comprising a needle member (144) removably coupled to the fixed length portion (508) and the loop portion (532), the needle member (144) adapted to facilitate passing the adjustable flexible member construct through tissue.

8. The apparatus of claim 1, further comprising an anchor member (140) slidably coupled to the first passage portion (48).

9. The apparatus of claim 8, wherein the anchor member (140) comprises an aperture, the first passage portion (48) extending through the aperture such that the first and second ends (38, 42) of the second flexible member (18) are adjustable relative to the anchor member (140).

10. The apparatus of claim 1, wherein the first and second flexible members (14, 18) comprise a suture formed of a braided or woven structure.

## Patentansprüche

1. Vorrichtung zur Verwendung bei chirurgischer Implantation, umfassend:
ein erstes flexibles Element (14), das ein erstes Ende (26), ein zweites Ende (30) und einen ersten Körper (22), der sich dazwischen erstreckt, aufweist, wobei der erste Körper (22) einen ersten Durchgangsabschnitt (48) definiert;
ein zweites flexibles Element (18), das ein erstes Ende (38), ein zweites Ende (42) und einen zweiten Körper (34), der sich dazwischen erstreckt, aufweist, wobei der zweite Körper (34) einen zweiten und einen dritten Durchgangsabschnitt (52, 52a) definiert;
wobei das erste Ende (26) des ersten flexiblen Elements (14) in den zweiten Durchgangsabschnitt (52) hinein und durch diesen hindurch verläuft, sodass sich das erste Ende (26) nach außerhalb des zweiten Durchgangsabschnitts (52) erstreckt, und das zweite Ende (30) des ersten flexiblen Elements (14) in den dritten Durchgangsabschnitt (52A) hinein und durch diesen hindurch verläuft, sodass sich das zweite Ende nach außerhalb des dritten Durchgangsabschnitts (52A) erstreckt; und
wobei das erste und das zweite Ende (38, 42) des zweiten flexiblen Elements in den ersten Durchgangsabschnitt (48) des ersten flexiblen Elements (14) und durch diesen hindurch verläuft, um ein selbstverriegelndes einstellbares flexibles Elementkonstrukt zu bilden, die Vorrichtung ferner umfassend den zweiten und den dritten Durchgangsabschnitt (52, 52A) umfasst, die durch einen Abschnitt (508) fester Länge des Körpers des zweiten flexiblen Elements (18) voneinander beabstandet sind.

2. Vorrichtung nach Anspruch 1, wobei sich das erste und das zweite Ende (38, 42) des zweiten flexiblen Elements (18) in den ersten Durchgangsabschnitt (48) hinein und durch diesen hindurch in entgegengesetzten Richtungen erstrecken, sodass sich das erste Ende (38) des zweiten flexiblen Elements (18) von einem Ende des ersten Durchgangsabschnitts (48) erstreckt und das zweite Ende (42) des zweiten flexiblen Elements (18) von dem anderen, gegenüberliegenden Ende des ersten Durchgangsabschnitts (48) erstreckt.

3. Vorrichtung nach Anspruch 1, wobei der Abschnitt (508) fester Länge des zweiten flexiblen Elements (18) in Zusammenwirken mit dem ersten und dem zweiten Ende (38, 42) des zweiten flexiblen Elements (18), das durch den ersten Durchgangsabschnitt (48) verläuft, eine selbstverriegelnde einstellbare Schleife (500) bildet.

4. Vorrichtung nach Anspruch 1, ferner umfassend einen ersten Abschnitt (522) einstellbarer Länge, der durch erste Abschnitte des ersten und des zweiten flexiblen Elements (14, 18) gebildet ist, die sich zwischen dem ersten und dem zweiten Durchgangsabschnitt (48, 52) erstrecken, und einen zweiten Abschnitt (526) einstellbarer Länge, der durch separate zweite Abschnitte des ersten und des zweiten flexiblen Elements (14, 18) gebildet ist, die sich zwischen dem ersten und dem dritten Durchgangsabschnitt (48, 52A) erstrecken;
wobei ein Anwenden von Spannung auf das erste und das zweite Ende des ersten und des zweiten flexiblen Elements (26, 30, 38, 42) eine Größe des ersten und des zweiten Abschnitts (522, 526) einstellbarer Länge reduziert.

5. Vorrichtung nach Anspruch 1, wobei das einstellbare flexible Elementkonstrukt (580) konfiguriert ist, um unter Spannung automatisch zu verriegeln, um die reduzierte Größe des ersten und des zweiten Abschnitts (522, 526) einstellbarer Länge unter Spannung aufrechtzuerhalten, wenn kein Knoten vorhanden ist.

6. Vorrichtung nach Anspruch 4, wobei der erste Körper einen hohlen Innenraum definiert, wobei das erste Ende (26) des ersten flexiblen Elements in den hohlen Innenraum des ersten Körpers in der Nähe des zweiten Endes (30) des ersten flexiblen Elements übergeht, und wobei das zweite Ende (30) des ersten flexiblen Elements in den hohlen Innenraum des ersten Körpers in der Nähe des ersten Endes (26) des ersten flexiblen Elements (14) übergeht, um einen Schlaufenabschnitt (532) zu bilden, der sich als der erste und der zweite einstellbare Längenabschnitt (522, 526) von einer gegenüberliegenden Seite (512) des zweiten und des dritten Durchgangsabschnitts (52, 32A) erstreckt.

7. Vorrichtung nach Anspruch 6, ferner umfassend ein Nadelelement (144), das abnehmbar mit dem Abschnitt (508) fester Länge und dem Schlaufenabschnitt (532) gekoppelt ist, wobei das Nadelelement (144) angepasst ist, um ein Verlaufen des einstellbaren flexiblen Elementkonstrukts durch Gewebe zu ermöglichen.

8. Vorrichtung nach Anspruch 1, ferner umfassend ein Verankerungselement (140), das gleitfähig mit dem ersten Durchgangsabschnitt (48) gekoppelt ist.

9. Vorrichtung nach Anspruch 8, wobei das Verankerungselement (140) eine Öffnung umfasst, wobei sich der erste Durchgangsabschnitt (48) durch die Öffnung erstreckt, sodass das erste und das zweite Ende (38, 42) des zweiten flexiblen Elements (18) in Bezug auf das Verankerungselement (140) einstellbar sind.

10. Vorrichtung nach Anspruch 1, wobei das erste und das zweite flexible Element (14, 18) eine Naht umfassen, die aus einer geflochtenen oder gewebten Struktur gebildet ist.

## Revendications

1. Appareil destiné à être utilisé dans une implantation chirurgicale, comprenant :
un premier élément flexible (14) ayant une première extrémité (26), une seconde extrémité (30) et un premier corps (22) s'étendant entre celles-ci, le premier corps (22) définissant une première partie de passage (48) ;
un deuxième élément flexible (18) ayant une première extrémité (38), une seconde extrémité (42) et un deuxième corps (34) s'étendant entre celles-ci, le deuxième corps (34) définissant des deuxième et troisième parties de passage (52, 52a),
la première extrémité (26) du premier élément flexible (14) passant dans et à travers la deuxième partie de passage (52) de telle sorte que la première extrémité (26) s'étende à l'extérieur de la deuxième partie de passage (52), et la seconde extrémité (30) du premier élément flexible (14) passant dans et à travers la troisième partie de passage (52A) de telle sorte que la seconde extrémité s'étende à l'extérieur de la troisième partie de passage (52A) ; et
les première et seconde extrémités (38, 42) du deuxième élément flexible passant dans et à travers la première partie de passage (48) du premier élément flexible (14) pour former une construction d'élément flexible réglable autobloquant, l'appareil comprenant en outre les deuxième et troisième parties de passage (52, 52A) espacées l'une de l'autre par une partie de longueur fixe (508) du corps du deuxième élément flexible (18).

2. Appareil selon la revendication 1, dans lequel les première et seconde extrémités (38, 42) du deuxième élément flexible (18) s'étendent dans et à travers la première partie de passage (48) dans des directions opposées de telle sorte que la première extrémité (38) du deuxième élément flexible (18) s'étende depuis une extrémité de la première partie de passage (48) et que la seconde extrémité (42) du deuxième élément flexible (18) s'étende depuis l'autre extrémité opposée de la première partie de passage (48).

3. Appareil selon la revendication 1, dans lequel la partie de longueur fixe (508) du deuxième élément flexible (18) en coopération avec les première et seconde extrémités (38, 42) du deuxième élément flexible (18) passant à travers la première partie de passage (48) forme une boucle réglable autobloquante (500).

4. Appareil selon la revendication 1, comprenant en outre une première partie de longueur réglable (522) formée par des premières parties des premier et deuxième éléments flexibles (14, 18) s'étendant entre les première et deuxième parties de passage (48, 52), et une deuxième partie de longueur réglable (526) formée par des deuxièmes parties séparées des premier et deuxième éléments flexibles (14, 18) s'étendant entre les première et troisième parties de passage (48, 52A) ;
dans lequel l'application d'une tension aux première et seconde extrémités des premier et deuxième éléments flexibles (26, 30, 38, 42) réduit une taille des première et deuxième parties de longueur réglable (522, 526).

5. Appareil selon la revendication 1, dans lequel la construction d'élément flexible réglable (580) est configurée pour se verrouiller automatiquement sous tension pour maintenir la taille réduite des première et deuxième parties de longueur réglable (522, 526) sous tension en l'absence de nœud.

6. Appareil selon la revendication 4, dans lequel le premier corps définit un intérieur creux, dans lequel la première extrémité (26) du premier élément flexible passe dans l'intérieur creux du premier corps à proximité de la seconde extrémité (30) du premier élément flexible, et dans lequel la seconde extrémité (30) du premier élément flexible passe dans l'intérieur creux du premier corps à proximité de la première extrémité (26) du premier élément flexible (14) pour former une partie de boucle (532) s'étendant à partir d'un côté opposé (512) des deuxième et troisième parties de passage (52, 32A) en tant que première et deuxième parties de longueur réglable (522, 526).

7. Appareil selon la revendication 6, comprenant en outre un élément d'aiguille (144) couplé de manière amovible à la partie de longueur fixe (508) et à la partie de boucle (532), l'élément d'aiguille (144) étant adapté pour faciliter le passage de la construction d'élément flexible réglable à travers un tissu.

8. Appareil selon la revendication 1, comprenant en outre un élément d'ancrage (140) couplé de manière coulissante à la première partie de passage (48).

9. Appareil selon la revendication 8, dans lequel l'élément d'ancrage (140) comprend une ouverture, la première partie de passage (48) s'étendant à travers l'ouverture de telle sorte que les première et seconde extrémités (38, 42) du deuxième élément flexible (18) soient réglables par rapport à l'élément d'ancrage (140).

10. Appareil selon la revendication 1, dans lequel les premier et deuxième éléments flexibles (14, 18) comprennent une suture formée d'une structure tressée ou tissée.
